(19) Europäisches Patentamt
European Patent Office
Office européen des brevets

(11) Veröffentlichungsnummer: **0 146 498**
**B1**

(12) # EUROPÄISCHE PATENTSCHRIFT

(45) Veröffentlichungstag der Patentschrift: **08.04.87**

(51) Int. Cl.⁴: **C 08 G 59/40**

(21) Anmeldenummer: **84810546.6**

(22) Anmeldetag: **09.11.84**

(54) **Härtbare Gemische, enthaltend ein Epoxidharz, ein Imid und einen Härtungskatalysator.**

(30) Priorität: **15.11.83 CH 6137/83**

(43) Veröffentlichungstag der Anmeldung: **26.06.85 Patentblatt 85/26**

(45) Bekanntmachung des Hinweises auf die Patenterteilung: **08.04.87 Patentblatt 87/15**

(84) Benannte Vertragsstaaten: **CH DE FR GB IT LI NL**

(56) Entgegenhaltungen:
**EP-A-0 032 745**
**FR-A-1 308 211**
**FR-A-2 355 047**
**FR-A-2 355 049**

(73) Patentinhaber: **CIBA- GEIGY AG, Klybeckstrasse 141, CH- 4002 Basel (CH)**

(72) Erfinder: **Schmid, Rolf, Dr., Buhnenstock 14, CH- 3150 Schwarzenburg (CH)**
Erfinder: **Renner, Alfred, Dr., Marcoup 2, CH- 3280 Muntelier (CH)**
Erfinder: **Stauffer, Werner, Av. Jean- Marie Musy 6, CH- 1700 Fribourg (CH)**
Erfinder: **Fischer, Michael, Dr., Allmendstrasse, CH- 1712 Tafers (CH)**

EP 0 146 498 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Vorliegende Erfindung betrifft heisshärtbare Gemische, die ein Epoxidharz, ein allyl- oder methallylsubstituiertes Imid und einen Härtungskatalysator enthalten, und die durch Härtung aus diesen Gemischen erhaltenen Formstoffe, Beschichtungen oder Verklebungen.

Aus der DE-OS 27 26 821 sind heisshärtbare Mischungen aus Di- oder Polymaleinimiden, mindestens eine Allylgruppe aufweisenden Epoxidverbindungen und gegebenenfalls Härtungsmitteln für Epoxidharze und/ oder Härtungsbeschleunigern bekannt. Die DE-OS 27 26 846 offenbart heisshärtbare Mischungen, die Di- oder Polymaleinimide, Alkenylphenole und/oder Alkenylphenoläther, mindestens eine Alkylgruppe enthaltende Epoxidverbindungen und gegebenenfalls Härtungsbeschleuniger enthalten. Diese vorbekannten härtbaren Mischungen sind verarbeitungstechnisch nicht unproblematisch, da zur Herstellung von homogenen Mischungen entweder ein organisches Lösungsmittel verwendet werden muss oder beim Arbeiten ohne Lösungsmittel diese Mischungen bei relativ hohen Temperaturen aufgeschmolzen werden müssen. Ausserdem lässt bei den aus diesen Mischungen hergestellten Formstoffen die Alterungsbeständigkeit in der Wärme zu wünschen übrig.

Es wurde nun gefunden, dass sich die oben genannten Nachteile durch Verwendung von bestimmten, allyl- oder methallylsubstituierten Imiden im Gemisch mit Epoxidharzen und Härtungskatalysatoren vermeiden lassen.

Gegenstand der Erfindung ist somit ein lagerstabiles, heisshärtbares Gemisch enthaltend

(a) 5 bis 95 Gewichtsteile eines Epoxidharzes mit durchschnittlich mehr als einer Epoxidgruppe im Molekül,

(b) 95 bis 5 Gewichtsteile eines allyl- oder methallylsubstituierten Imids der Formel I

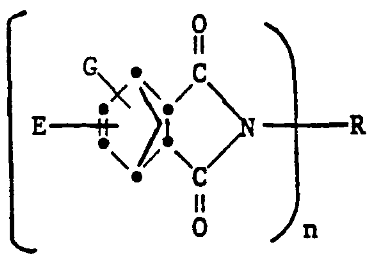

(I),

worin E Allyl oder Methallyl, G Wasserstoff oder Methyl, und n 1 oder 2 bedeuten und R, falls n 1 bedeutet, für Wasserstoff, Alkyl mit 1-12 G-Atomen, Alkenyl mit 3-6 G-Atomen, Cycloalkyl mit 5-8 C-Atomen, Aryl mit 6-10 C-Atomen, Hydroxyphenyl oder Benzyl oder, falls n 2 bedeutet, für $-C_mH_{2m}$ - mit m 2-20, Arylen mit 6-10 C-Atomen oder für eine Gruppe der Formel II

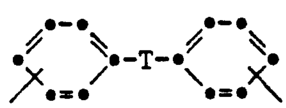

(II),

worin T Methylen, Isopropyliden, CO, O, S oder $SO_2$ bedeutet, steht, und wobei im Gemisch die Summe aus (a) und (b) 100 Gewichtsteile beträgt, und

(c) 1 bis 15 Gewichtsteile, bezogen auf 100 Gewichtsteile des Gemisches aus (a) und (b), eines Härtungskatalysators. Vorzugsweise bedeutet G in Formel I ein Wasserstoffatom.

Vorzugsweise enthält das erfindungsgemässe Gemisch 20 bis 70 Gewichtsteile, insbesondere 30 bis 60 Gewichtsteile, des Epoxidharzes (a), 80-30 Gewichsteile, insbesondere 70 bis 40 Gewichtsteile, des Imids (b) und 3 bis 10 Gewichtsteile, insbesondere 4-7 Gewichtsteile, des Härtungskatalysators (c).

In den erfindungsgemässen Mischungen lassen sich als Epoxidharz (a) im Prinzip alle Typen vom Epoxidharzen einsetzen, wie beispielsweise solche mit mindestens zwei direkt an ein Sauerstoff-, Stickstoff- oder Schwefelatom bzw. -atome gebundenen Glycidyl- oder β-Methylglycidylgruppen. Als Beispiele solcher Epoxidharze seien die Polyglycidyl- und Poly-(B-methylglycidyl)-ester genannt, die man durch Umsetzung einer zwei oder mehr Carbonsäuregruppen pro Molekül enthaltenden Verbindung mit Epichlorhydrin, Glycerindichlorhydrin oder β-Methylepichlorhydrin im Gegenwart von Alkali erhalten kann. Solche Polyglycidylester können sich von aliphatischen Polycarbonsäuren, z.B. Bernsteinsäure, Glutarsäure, Adipinsäure, Azelainsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, sowie von aromatischen Polycarbonsäuren, wie Phthalsäure, Isophthalsäure und Terephthalsäure, ableiten.

Weitere Beispiele sind Polyglycidyl- und Poly-(β-methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Aether lassen sich aus acyclischen Alkoholen, wie Aethylenglykol, Diäthylenglykol und höheren Poly-(oxyäthylen)-glykolen, Propan-1,2-diol und Poly-(oxypropylen)-glykolen, Propan-1,3-diol, Butan-1,4-diol, Hexam-1,6-diol, Hexam-2,4,6-triol,

Glycerin, 1,1,1-Trimethylolpropan, Pentaerythrit, Sorbit und Polyepichlorhydrinen, aus cycloaliphatischen Alkoholen, wie Resorcit, Chinit, Bis-(4-hydroxy-cyclohexyl)-methan, 2,2-Bis-(4-hydroxycyclo-hexyl)-propan und 1,1-Bis(hydroxymethyl)-3-cyclohexen, und aus Alkoholen mit aromatischen Kernen, wie N,N-Bis-(2-hydroxyäthyl)-amilin und p,p'-Bis-(2-hydroxyäthylamino)-diphenylmethan, herstellen. Man kann sie ferner aus einkernigen Phenolen, wie Resorcin und Hydrochinon, und mehrkernigen Phenolen, wie Bis-(4-hydroxyphenyl)-methan, 4,4'-Di-hydroxydiphenyl, Bis-(4-hydroxyphenyl)-sulfon, 1,1,2,2-Tetrakis-(4-hydroxyphenyl)-äthan, 2,2-Bis-(4-hydroxyphenyl)-propan und 2,2-Bis-(3,5-dibrom-4-hydroxyphenyl)-propan, sowie aus von Aldehyden, wie Formaldehyd, Acetaldehyd, Chloral und Furfurol mit Phenolen, wie Phenol selbst und durch Chloratome oder Alkylgruppen mit jeweils bis zu 9 Kohlenstoffatomen ringsubstituiertem Phenol, wie 4-Chlorphenol, 2-Methylphenol und 4-tert.-Butylphenol, gebildeten Novolaken herstellen.

Poly-(N-glycidyl)-verbindungen umfassen beispielsweise solche, die durch Dehydrochlorierung der Umsetzungsprodukte von Epichlorhydrin mit mindestens zwei Aminowasserstoffatome enthaltenden Aminen, wie Anilin, n-Butylamin, Bis-(4-aminophenyl)-methan, m-Xylylendiamin und Bis-(4-methylaminophenyl)-methan, erhalten werden, Triglycidylisocyanurat sowie N,N'-Diglycidylderivate von cyclischen Alkylenharnstoffen, wie Aethylenharnstoff und 1,3-Propylenharnstoff, und Hydantoinen, wie 5,5-Dimethylhydantoin.

Poly-(S-glycidyl)-verbindungen sind zum Beispiel die Di-S-glycidylderivate von Dithiolen, wie Aethan-1,2-dithiol und Bis-(4-mercapto-methylphenyl)-äther.

Ferner sind beispielsweise auch Epoxidharze geeignet, in welchen die Glycidylgruppen an Heteroatome verschiedener Art gebunden sind, beispielsweise das N,N,O-Triglycidylderivat des 4-Aminophenols, der Glycidyläther/Glycidylester der Salicylsäure, N-Glycidyl-N'-(2-glycidyl-oxypropyl)-5,5-dimethylhydantoin und 2-Glycidyloxy-1,3-bis-(5,5-di-methyl-1-glycidylhydantoin-3-yl)-propan.

Für die erfindungsgemässen heisshärtbaren Mischungen kommen auch die cycloaliphatischen Epoxidharze, worin die Epoxygruppe Teil des aliphatischen Ringsystems ist, in Betracht, wie beispielsweise Bis-(2,3-epoxycyclopentyl)-äther, 2,3-Epoxycyclopentyl-glycidyläther und 1,2-Bis-(2,3-epoxycyclopentyloxy)-äthan, doch sind sie weniger gut geeignet.

Gewünschtenfalls kann ein Gemisch aus Epoxidharzen verwendet werden.

Bevorzugte Epoxidharze sind Polyglycidyläther und Poly-(N-glycidyl)derivate aromatischer Amine. Speziell bevorzugte Harze sind die Polyglycidyläther von mehrkernigen Phenolen, wie 2,2-Bis-(4-hydroxyphenyl)-propan, Bis-(4-hydroxyphenyl)-methan oder aus Formaldehyd und Phenol oder durch ein Chloratom oder ein Alkyl mit 1 bis 4 C-Atomen ringsubstituierten Phenol gebildeten Novolak mit einem 1,2-Epoxidgehalt von mindestens 0,5 Aequivalenten pro Kilogramm.

Als Imide (b) der Formel I können in den erfindungsgemässen Mischungen beispielsweise Allyl-bicyclo[2.2.1]hept-5-en-2,3-dicarbonsäure-imid, Allyl- bicyclo[2.2.1]hept-5-en-2,3-dicarbonsäure-N-methylimid, Allyl-bicyclo[2.2.1]hept-5-en-2,3-dicarbonsäure-N-allylimid,Allyl-bicyclo[2.2.1]hept-5-en-2,3-dicarbonsäure-N-(2-äthyl-hexyl)-imid, Allyl-bicyclo[2.2.1]hept-5-en-2,3-dicarbonsäure-N-cyclohexylimid, Allyl-bicyclo[2.2.1]hept-5-en-2,3-dicarbonsäure-N-phenylimid, Allyl-bicyclo[2.2.1]hept-5-en-2,3-dicarbonsäure-N-benzylimid, N,N'-Aethylen-bis-(allyl-bicyclo[2.2.1]hept-5-en-2,3-dicarbonsäureimid), N,N'-Hexamethylen-bis-(allyl-bicyclo[2.2.1]hept-5-en-2,3-dicarbonsäureimid), N,N'-Dodecamethylen-bis(allyl-bicyclo[2.2.1]hept-5-en-2,3-dicarbonsäureimid), Bis-[4-(allyl-bicyclo[2.2.1]hept-5-en-2,3-dicarbonsäure-imidophenyl)-methan], Bis-[4-(methallyl-bicyclo[2.2.1]hept-5-en-2,3-dicarbonsäureimidophenyl)-methan], N,N'-Phenylen-bis-(allyl-bicyclo-[2.2.1]hept-5-en-2,3-dicarbonsäureimid), Bis-[4-(allyl-bicyclo[2.2.1]-hept-5-en-2,3-dicarbonsäureimidophenyl)-äther] und Bis-[4-(allyl-bi-cyclo[2.2.1]hept-5-en-2,3-dicarbonsäureimidophenyl)-sulfon] enthalten sein.

Vorzugsweise setzt man in den erfindungsgemässen Mischungen als Imide (b) solche der Formel I ein, worin E Allyl und G Wasserstoff bedeuten und R, falls n = 1 ist, für Wasserstoff, Alkyl mit 1-8 C-Atomen, Allyl, Cyclohexyl, Phenyl Hydroxyphenyl oder Benzyl steht, oder falls n = 2 ist, für $-(CH_2)_m-$ mit m = 2-12, m- oder p-Phenylen oder für eine Gruppe der Formel II steht, worin T die Methylengruppe, O oder $SO_2$ bedeutet.

Besonders bevorzugt sind Verbindungen der Formel I, worin E die Allylgruppe, G Wasserstoff, n die Zahl 2 und R $-(CH_2)_2-$, $-(CH_2)_6-$

oder 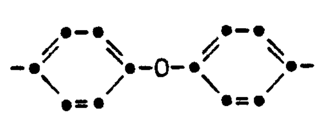 und insbesondere 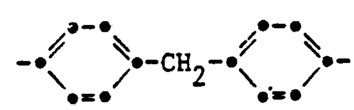

bedeuten.

Gewünschtenfalls kann auch ein Gemisch von Imiden der Formel I verwendet werden.

Die in den erfindungsgemässen Gemischen enthaltenen Imide (b) können in bekannter Weise durch Umsetzung eines Anhydrids der Formel III

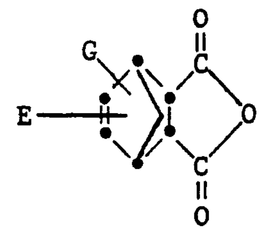

(III)

mit einem Mono- bzw. Diamin der Formel IV

$$(H_2N)_n\!-\!R \qquad (IV),$$

worin E, G, R und n die unter Formel I angegebene Bedeutung haben, bei erhöhter Temperatur und unter Abdestillieren des bei der Reaktion entstehenden Wassers hergestellt werden. Sofern es sich bei den Verbindungen der Formel IV um Ammoniak oder niedrigsiedende Monoamine handelt, ist ein Ueberschuss dieser Reaktanden zu empfehlen. Diamine sind vorteilhaft in stöchiometrischem Verhältnis einzusetzen. Die Umsetzung kann ohne Lösungsmittel oder in Gegenwart eines inerten Lösungsmittels, das für die azeotrope Entfernung des Wassers verwendbar ist (Schleppmittel) erfolgen. Die Temperatur der Umsetzung kann zwischen 100 und 250° C liegen. Bevorzugt werden die Imide der Formel I in der Schmelze bei einem Druck von höchstens 4500 Pa bei Temperaturen zwischen 130 und 220° C, insbesondere 180 und 220° C, hergestellt.

Die Anhydride der Formel III können gemäss dem in der US Fatentschrift 3 105 839 beschriebenen Verfahren hergestellt werden, indem man Natrium-Cyclopentadienid mit einem Allyl- oder Methallylhalogenid umsetzt, worauf sich eine Diels-Alder-Reaktion mit Maleinsäureanhydrid anschliesst. Obgleich in der US-Patentschrift angegeben wird, dass die Allylgruppe in 7-Stellung des bicyclischen Systems gebunden ist, zeigen neuere Untersuchungen, dass eine isomere Mischung in Bezug auf die Stellung der Allylgruppe und auch auf die Endo- und Exokonfiguration des Anhydridteils gebildet wird. Die isomeren Komponenten können nur durch präparative Gaschromatographie getrennt werden.

Als Härtungskatalysatoren (c) eignen sich für die erfindungsgemässen Gemische die üblichen zur Härtung von Epoxidharzen verwendbaren Katalysatoren, wie zum Beispiel Imidazol und dessen durch Alkyl, Alkenyl, Phenyl oder Benzyl substituierte Derivate, wie 1-Methylimidazol, 2-Aethyl-4-methylimidazol, 2-Vinylimidazol, 2-Phenylimidazol oder 2-Phenyl-4-methylimidazol, 1-(3-Aminopropyl)-imidazol, N-acylsubstituierte Imidazole, wie beispielsweise 1-(2,4,6-Trimethylbenzoyl)-2-phenylimidazol, 1-(2,6-Dichlorbenzoyl)-2-methylimidazol, 1-(2,6-Dichlorbenzoyl)-phenylimidazol, 1-(2,6-Dichlorbenzoyl)-2-äthyl-4-methylmidazol, 1-(2,6-Dichlorbenzoyl)-4-phenylimidazol, 1-(2-Chlor-6-nitrobenzoyl)-2-phenylimidazol, 1-(2-Chlor-6-nitrobenzoyl)-2-äthyl-imidazol, 1-Pentachlorbenzoyl-2-methylimidazol, 1-Pentachlorbenzoyl-2-phenylimidazol, 1-Propenyl-2-phepylimidazol oder 1-Propenyl-4-phenylimidazol, die zum Beispiel in der DE-OS 32 46 072 und in dem JP-Patent 743 212 offenbart werden, tertiäre Amine, wie Diäthylaminopropylamin, Dimethylaminopropylamin, Diäthylaminoäthylamin, Dimethylaminobenzylamin oder 1,6-Bis-(dimethylamino)-hexan, sowie Additionsprodukte aus Dimethylaminoalkylaminen und Glycidyläthern aliphatischer Alkohole oder von Phenolen. Solche Additionsprodukte entsprechend vorzugsweise der Formel V

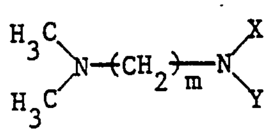

(V)

worin m für eine Zahl von 3 bis 10 steht,
X eine Gruppierung der Formel

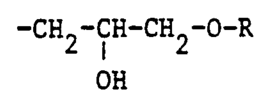

bedeutet, worin R ein Alkyl mit 4 bis 10 C-Atomen oder ein unsubstituiertes oder durch Chlor, Brom, Alkyl oder Alkoxy mit je 1 bis 3 C-Atomen substituiertes Phenyl bedeutet, und Y für ein Wasserstoffatom steht oder die Bedeutung von X hat. Additionsprodukte der oben angegebenen Formel sind zum Beispiel in der GB-FS 1 169 990 und in der US-PS 3 332 997 beschrieben.

Ferner können als Härtungskatalysatoren beispielsweise auch Verbindungen der Formeln
$(CH_3)_2N\text{-}(CH_2)_3\text{-}NH(CH_2)_3\text{-}NH_2$,
$(CH_3)_2N(CH_2)_3NH(CH_2)_2\text{-}CN$ oder
$(CH_3)_2N\text{-}(CH_2)_3\text{-}N(CH_2CH_2CN)_2$

eingesetzt werden, sowie Piperazin, Dicyandiamid, die bekannten $BF_3$-Komplexverbindungen, wie zum Beispiel Bortrifluorid-monoäthylaminkomplex, Addukte aus 1 Mol 1-(3-Aminopropyl)-imidazol und 1 oder 2 Mol Glycidyläther aliphatischer Alkohole oder von Phenolen oder Addukte aus 1 Mol 2-Aethyl-4-methylimidazol, 2-Methylimidazol oder Imidazol und 1 Mol Glycidyläther aliphatischer Alkohole oder eines Phenols. Diese Addukte können ebenfalls nach dem in der GB-PS 1 169 990 beschriebenen Verfahren hergestellt werden.

Vorzugsweise verwendet man als Härtungskatalysator Imidazol und dessen substituierte Derivate, insbesondere die N-acylsubstituierten Imidazole.

Die erfindungsgemässen Gemische bieten den Vorteil, dass sie ohne Lösungsmittel verarbeitet werden können. Sie weisen eine günstige Verarbeitbarkeit auf, da sie relativ niedrigviskos sind und bei Verarbeitungstemperaturen bis zu 100°C eine gute Latenz aufweisen. Oberhalb von 100°C gelieren die erfindungsgemässen Gemische dann rasch.

Die erfindungsgemässen Gemische können vielseitig angewendet werden und eignen sich beispielsweise als Giessharze, Laminier- oder Tränkharze, Formmassen, Dichtungsmassen, Einbettungs- und Isoliermassen für die Elektrotechnik und vorzugsweise als Klebstoffe und als Matrixharze für Verbundstoffe, insbesondere zur Herstellung von faserverstärkten Kunststoffen.

Gewünschtenfalls, insbesondere bei der Mitverwendung von Modifizierungsmitteln, können die erfindungsgemässen Gemische in einem organischen Lösungsmittel, wie Toluol, Xylol, Methyläthylketon, Methylenchlorid, Aethylenglykolmonoalkyl- und -dialkyläthern mit 1-4 C-Atomen in der bzw. den Alkylgruppen oder einem ähnlichen, in der Lackindustrie üblichen Lösungsmittel gelöst werden. Solche Lösungen eignen sich vor allem als Imprägnierungsmittel oder Beschichtungsmittel.

Die erfindungsgemässen härtbaren Mischungen können ferner vor der Härtung in irgendeiner Phase mit üblichen Modifizierungsmitteln, wie Streck-, Füll- und Verstärkungsmitteln, Pigmenten, Farbstoffen, organischen Lösungsmitteln, Weichmachern, Verlaufmitteln, Thixotropiermitteln, flammhemmenden Stoffen oder Formtrennmitteln, versetzt werden. Als Streckmittel, Verstärkungsmittel, Füllmittel und Pigmente, die in den erfindungsgemässen härtbaren Mischungen eingesetzt werden können, seien z.B. genannt: Glasfasern, Asbestfasern, Borfasern, Kohlenstofffasern, Fasern aus aromatischen Polyamiden, Quarzmehl, mineralische Silikate, wie Glimmer, Asbestmehl, Schiefermehl, Kaolin, Aluminiumoxid, Kreidemehl, Antimontrioxid, Bentone, Kieselsäureaerogel, Lithopone, Schwerspat, Titandioxid, Russ, Graphit, Oxidfarben, wie Eisenoxid, oder Metallpulver, wie Aluminiumpulver oder Eisenpulver, insbesondere Zusätze von 2-10 Gewichts-% an hochfeinem $Al_2O_3$ oder Kieselsäureaerogel.

Als Verlaufmittel beim Einsatz der härtbaren Mischungen speziell im Oberflächenschutz, kann man z.B. Silikone, flüssige Acrylharze, Celluloseacetobutyrat, Polyvinylbutyral, Wachse, Stearate etc. (welche z.T. auch als Formtrennmittel Anwendung finden) zusetzen.

Als Modifiziermittel können den härtbaren Mischungen zwecks Erhöhung von Flexibilität und Bruchzähigkeit Polymere oder Präpolymere, wie Polysulfone, Polyamide, Polyäthersulfone, Polycarbonate oder Polyamid-imid Copolymere zugesetzt werden.

Die erfindungsgemässen Gemische werden vorzugsweise gehärtet, indem man sie auf eine Temperatur im Bereich von 120 bis 250°C, insbesondere 180° bis 220°C erhitzt. Man kann die Härtung in bekannter Weise auch zwei- oder mehrstufig durchführen, wobei man die erste Härtungsstufe bei niedriger Temperatur und die Nachhärtung bei höherer Temperatur durchführt.

Gegenstand vorliegender Erfindung sind somit auch die aus den härtbaren erfindungsgemässen Gemischen durch Härtung erhaltenen Formstoffe, Beschichtungen und Verklebungen. Die erfindungsgemässen Formstoffe zeichnen sich im allgemeinen durch relativ hohe Glasumwandlungstemperaturen bei gleichzeitig hohen mechanischen Festigkeiten, hohe Feuchtigkeitsstabilitäten und insbesondere durch eine ausgezeichnete Dauerwärmebeständigkeit aus und eignen sich daher besonders zur Herstellung von Prepregs und hochwertigen Verbundstoffen.

Die nachfolgenden Beispiele erläutern die Erfindung näher. Die in den Beispielen verwendeten Imide (b) der Formel I werden wie folgt hergestellt:

Imid A: N,N''-Hexamethylen-bis-(allyl-bicyclo[2.2.1]hept-5-en-2,3-dicarbonsäureimid)

Eine Mischung aus 204 g Allyl-bicyclo[2.2.1]hept-5-en-2,3-dicarbon-säureanhydrid und 58 g Hexamethylendiamin wird im Verlauf von 3 Stunden unter Rühren am absteigenden Kühler auf 165°C erhitzt. Danach wird der Druck auf 1866 Pa reduziert und die Mischung eine weitere Stunde bei 175°C gerührt. Man erhält 235 g eines bernsteinfarbenen Harzes, das bei Raumtemperatur gerade noch flüssig ist.

| Analyse: | % C | % H | % N |
|---|---|---|---|
| berechnet für $C_{30}H_{36}N_2O_4$: | 73,74 | 7,43 | 5,73 |
| gefunden: | 73,4 | 7,4 | 5,5 |

Imid B: N,N'-Aethylen-bis-(allyl-bicyclo[2.2.1]hept-5-en-2,3-dicarbonsäureimid).

Man legt 204 g Allyl-bicyclo[2.2.1]hept-5-en-2,3-dicarbonsäureanhydrid vor und tropft unter Rühren 30 g Aethylendiamin ein. Die Temperatur steigt bis auf 130°C an. Man steigert die Temperatur auf 180°C; hierbei destillierten 14 ml Wasser ab. Danach erhitzt man weitere 2 Stunden auf 200°C unter einem Druck von 9,3 Pa. Man erhält 210 g eines gelben, bei Raumtemperatur festen Harzes mit einem Erweichungspunkt von 56°C, gemessen auf der Kofler Heizbank.

| Analyse: | % C | H % | % N |
|---|---|---|---|
| berechnet für $C_{26}H_{28}N_2O_4$: | 72,20 | 6,53 | 6,48 |
| gefunden: | 71,7 | 6,5 | 6,4. |

Imid C: Bis-[4-(allyl-bicyclo[2.2.1]hept-5-en-2,3-dicarbonsäureimidophenyl)]-methan

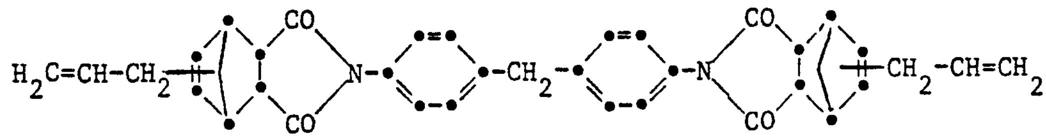

204 g Allyl-bicyclo[2.2.1]hept-5-en-2,3-dicarbonsäureanhydrid und 99 g 4,4'-Diaminophenylmethan werden in Vakuum auf 200°C erhitzt und 1 Stunde gehalten. Man erhält 280 g eines braunen Festharzes mit einem Erweichungspunkt von 104°C, einer Viskosität von 0,425 Pa.s bei 200°C und einer Säurezahl von 0.

| Analyse: | % C | % H | % N |
|---|---|---|---|
| berechnet für $C_{37}H_{34}N_2O_4$: | 77,87 | 6.01 | 4,91 |
| gefunden: | 78,2 | 6,1 | 5,0 |

In den Beispielen werden als Härtungskatalysatoren folgende Verbindungen eingesetzt:

Imid D: Bis-[4-(methallyl-methyl-bicyclo[2.2.1]hept-5-en-2,3-dicarbonsäureimidophenyl)]-methan

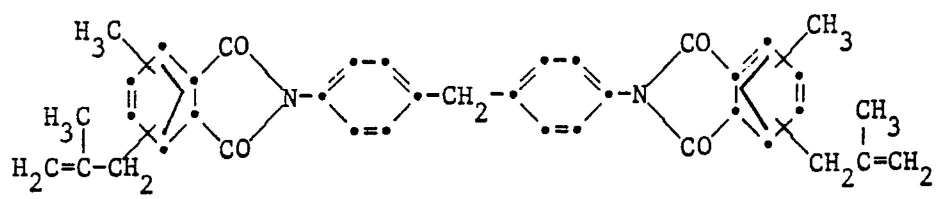

Man erhitzt 116 g Methallyl-methyl-bicyclo[2.2.1]hept-5-en-2,3-di-carbonsäureanhydrid und 49,5 g 4,4'-Diaminodiphenylmethan unter Rühren in einer $N_2$-Atmosphäre auf 200°C. Es destillieren 9 $cm^3$ Wasser ab. Im Verlauf von 35 Minuten bei 200°C steigt die Glasumwandlungstemperatur von 67,5 auf 78,5°C. Ausbeute: 155 g (99 % der Theorie).

| Analyse: | berechnet: | gefunden: |
|---|---|---|
| % C | 78,57 | 77,41 |
| % H | 6,75 | 6,71 |
| % N | 4,47 | 4,38 |

Imid E: Allyl-bicylco[2.2.1]hept-5-en-2,3-dicarbonsäure-N-(4'-hydroxyphenyl)-imid

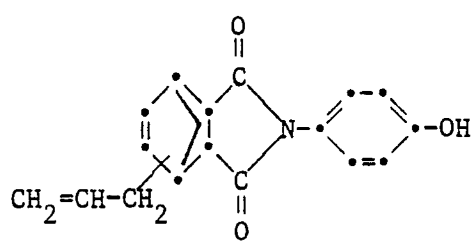

Man erhitzt 408 g eines Isomerengemisches von Allyl-bicyclo[2.2.1]hept-5-en-2,3-dicarbonsäureanhydrid mit 238,26 g 4-Aninophenol auf 200°C, erniedrigt den Druck auf 2,7 Pa und hält 1 Stunde unter diesen Bedingungen. Man erhält 535 g eines roten Festharzes (87,7 % der Theorie) mit einer Glasumwandlungstemperatur von 58°C.

| Analyse: | berechnet: | gefunden: |
|---|---|---|
| % C | 73,20 | 72,43 |
| % H | 5,90 | 6,05 |
| % N | 4,89 | 4,90 |
| % OH | 5,76 | 5,38 |

Katalysator I: 1-(2,4,6-Trimethylbenzoyl)-2-phenylimidazol, das wie folgt hergestellt wird:

144,2 g (1,00 Mol) 2-Phenylimidazol werden bei 90°C in 900 ml Toluol gelöst. Zu dieser Lösung fügt man 104,2 g (1,03 Mol) Triäthylamin zu und lässt bei 90°C eine Lösung von 182,6 g (1,00 Mol) 2,4,6-Trimethylbenzoylchlorid in 300 ml Toluol innerhalb 2 Stunden zutropfen, wobei sich Triäthylaminhydrochlorid abscheidet. Dieses wird bei Zimmertemperatur abgesaugt und mit Toluol ausgewaschen. Nach dem Einengen des Filtrats erhält man 296 g Rohprodukt, dessen Umkristallisation aus 580 ml Acetonitril insgesamt 227,3 g an 1-(2,4,6-Trimethylbenzoyl)-2-phenylimidazol (78,3 % d. Theorie) ergibt.

Katalysator II: 1-(2,4,6-Trimethylbenzoyl)-2-äthylimidazol, das analog Katalysator I unter Verwendung des 2-Aethylimidazols anstelle von 2-Phenylimidazol hergestellt wird.

Katalysator III: 1-(2,6-Dichlorbenzoyl)-2-äthylimidazol, hergestellt aus 2-Aethylimidazol und 2,6-Dichlorbenzoylchlorid analog Katalysator I.

Katalysator IV: 1-(2,6-Dichlorbenzoyl)-2-phenylimidazol, hergestellt aus 2-Phenylimidazol und 2,6-Dichlorbenzoylchlorid analog Katalysator I.

Katalysator V: 2-Aethyl-4-methylimidazol

Beispiel 1: 35 g eines Epoxidphenolnovolakharzes mit einem Epoxidgehalt von 5,6 -5,8 Aequivalenten/kg werden auf 100°C erwärmt und mit 15 g Imid A sowie 2,1 g Katalysator I gut gemischt. Die Mischung hat bei 120°C eine Viskosität von 48 mpa.s und eine Gelierzeit von 116 Minuten (min). Nach Entlüftung der Mischung und Härtung derselben in einer 4 mm dicken aluminumform (Anticorodal) während 2 Stunden (h) bei 150°C und 2 h bei 250°C werden homogene Formkörper mit folgenden Eigenschaften erhalten:

Glasumwandlungstemperatur Tg (gemessen mit TMA 3000* als Peak-Maximum der Eindringgeschwindigkeit) = 174°C

Biegefestigkeit nach ISO 178 (BF) bei 23°C = 120 MPa

Biegefestigkeit nach ISO 178 nach 10-tägiger Lagerung in $H_2O$ bei 85°C = 104 MPa

Randdehnung nach ISO 178 (RD) = 6,3 %

Bruchzähigkeit (BZ) (gemessen nach dem Double-Torsionstest) = 111 J/m$^2$

Zersetzungstemperatur (Tz) (maximale Verdampfungsgeschwindigkeit der flüchtigen Fragmente bei Erwärmung von 4°C/min) = 417°C

Zur Bestimmung der Bruchzähigkeit nach dem Double-Torsionstest gemäss Vorschrift von P.W.R. Beaumont und R.J.Young, beschrieben in "Journal of Materials Science, 10, 1334 (1975) und 11, 776 (1979), werden zwei Aluminium-Profile mit dem härtbaren Gemisch verklebt und die Verklebung wie oben angegeben gehärtet. Bei dieser Messmethode wird die Rissfortpflanzung in der Verklebung gemessen, d.h., aus der maximalen Last für die Rissfortpflanzung wird die Bruchenergie in J/m berechnet.

*) TMA 3000 = thermomechanischer Analysator 3000 der Firma Mettler AG, Greifensee, CH.

Beispiel 2: Bei Verwendung von 15 g Imid B und sonst gleicher Zusammensetzung und Verarbeitung wie in Beispiel 1 werden folgende Eigenschaften gemessen:.

| | |
|---|---|
| Viskosität bei 120°C | 77 mPa.s |
| Gelierzeit bei 120°C | 86 min |
| Tg | 192°C |
| BF | 107 MPa |
| BF nach 10 Tagen Lagerung in $H_2O$ bei 85°C | 102 MPa |
| RD | 5,0 % |
| Torsionsklebefestigkeit (Twist-o-Meter) (TF) bei 25°C | 102 MPa |
| Torsionsklebefestigkeit (Twist-o-Meter) (TF) bei 120°C | 63 MPa |
| BZ 70 | J/m$^2$ |

Zur Bestimmung der Torsionsklebefestigkeit werden gemäss Vorschrift für Messungen mit dem "Twist-o-Meter (Firma Epprecht, Instruments+ Controls, Bassersdorf, CH) Aluminiumzapfen verklebt. Dazu wird die härtbare Mischung unter Rühren auf 120°C erhitzt, bis man eine homogene, niederviskose Lösung erhält. Mit der auf Raumtemperatur abgekühlten Lösung werden 5 Verklebungen hergestellt, die dann durch Erhitzen während 2 Stunden auf 150°C una 2 Stunden 250°C gehärtet werden.

Beispiel 3: Bei Verwendung von 15 g Imid C und sonst gleicher Verarbeitung und Härtung wie in Beispiel 1 werden homogene Formkörper mit folgenden Eigenschaften erhalten:

| | | |
|---|---|---|
| Tg | | 212°C |
| BF | | 105 MPa |
| BF nach 10 Tagen Lagerung in $H_2O$ bei 85°C | | 99 MPa |
| RD | | 5,3 % |
| TF | bei 250°C | 104 MPa |
| TF | bei 120°C | 63 MPa |

Beispiel 4: Bei Verwendung von 30 g Imid A, 20 g des in Beispiel 1 verwendeten Epoxidphenolnovolaks und 1,2 g Katalysator 1 und Härtung während 2 h bei 150°C und 2 h bei 250°C werden an der härtbaren Mischung bzw. an den erhaltenen Formkörpern folgende Eigenschaften gemessen:

| | | |
|---|---|---|
| Viskosität bei 120°C | | 110 mPa.s |
| Gelierzeit bei 120°C | | 700 min |
| Tg | | 172°C |
| BF | | 110 MPa |
| RD | | 5,4 % |
| TF | bei 25°C | 109 MPa |
| | bei 120°C | 68 MPa |

Beispiel 5: Bei Verwendung von 50 g Epoxidphenolnovolak gemäss Beipiel 1,35 g Imid A, und 15 g Imid C und 3 g Katalysator 1 und sonst gleicher Verarbeitung und Härtung wie in Beispiel 4 werden folgende Eigenschaften gemessen:

| | | |
|---|---|---|
| Gelierzeit bei 120°C | | 120 min |
| Tg | | 175°C |
| BF | | 122 MPa |
| RD | | 6,0 % |
| TF | bei 25°C | 118 MPa |
| TF | bei 120°C | 78 MPa |
| BZ | | 78 J/m$^2$ |

Beispiel 6: Die aus den folgenden 3 Mischungen hergestellten Formstoffe werden einem Alterungstest

unterworfen:.

(1) 50 g Epoxidphenolnovolak gemäss Beispiel 1 + 50 g Imid A
(2) 50 g " " " " + 50 g Imid C
(3) 50 g " " " " + 25 g Imid C + 25 g Imid A

Die Mischungen werden mit je 3 g Katalysator 1 vermischt. Härtung der Mischungen: 2 h bei 150°C und 2 h bei 250°C.

Nach der Härtung werden an den Formkörpern folgende Werte gemessen:

| | (1) | (2) | (3) |
|---|---|---|---|
| BF [MPa] | 110 | 117 | 113 |
| Dehnung bei Bruch (ε) [%] | 4,7 | 5,8 | 4,9 |

nach 10-tägiger Lagerung in Wasser von 85°C werden folgende Gewichtsänderungen gemessen[%]:

| | (1) | (2) | (3) |
|---|---|---|---|
| | 1,90 | 2,05 | 2,00 |

nach 10-tägiger Lagerung in Luft bei 160°C werden folgende Werte erhalten:.

| | (1) | (2) | (3) |
|---|---|---|---|
| BF [MPa] | 111 | 106 | 113 |
| ε [%] | 4,2 | 5,3 | 5,3 |

nach 10-tägiger Lagerung in Luft bei 210°C werden folgende Werte erhalten:

| | (1) | (2) | (3) |
|---|---|---|---|
| BF [MPa] | 85 | 88 | 101 |
| ε [%] | 2,9 | 3,2 | 3,8 |

Die aus den erfindungsgemässen Mischungen erhaltenen Formstoffe sind wenig empfindlich gegenüber Feuchtigkeit und zeigen eine gute Stabilität bei der Alterung in heisser Luft.

Beispiel 7: 14 g des Epoxidharzes gemäss Beispiel 1 werden mit 6 g Imid C in der Wärme gemischt, dann auf etwa 100°C abgekühlt und mit 0,84 g Katalysator II vermischt. Die Mischung hat bei 160°C eine Gelierzeit von 8,4 Minuten und zeigt nach Härtung während 2 h bei 150°C und 2 h bei 250°C eine T von 170°C.

Beispiel 8: 14 g Bisphenol-A-diglycidyläther mit einem Epoxidäquivalentgewichts von 185, 6 g Imid C und 0,84 g Katalysator III werden wie in Beispiel 7 gemischt und gehärtet. Die Mischung hat bei 160°C eine Gelierzeit von 17,5 Minuten. Die $T_g$ der gehärteten Mischung beträgt 179°C.

Beispiel 9: 14 g N,N,N',N'-Tetraglycidyl-diaminodiphenylmethan werden mit 6 g Imid A in der Wärme gemischt und nach Abkühlen auf etwa 100°C mit 0,84 g Katalysator IV gemischt. Die Mischung hat bei 160°C eine Gelierzeit von 37,5 Minuten. Nach der Härtung gemäss Beispiel 7 beträgt die $T_h$ des Formkörpers 206°C.

Beispiel 10: 14 g eines Epoxidphenolnovolaks mit einem Epoxidäquivalentgewicht von 191 und einer Funktionalität von etwa 3,5 Epoxidgruppen pro Molekül werden in der Wärme mit 6 g Imid A gemischt und nach Abkühlen auf etwa 50°C mit 0,6 g Katalysator V gemischt. Die Mischung hat bei 160°C eine Gelierzeit von 1,2 Minuten. Nach Härtung der Mischung entsprechend Beispiel 7 weist der erhaltene Formkörper ein $T_g$ von 184°C auf.

Beispiel 11: 25 g Imid C, 25 g Imid A, 15 g Bisphenol-A-diglycidyläther (BADG) mit einem Epoxidäquivalentengewicht von 185, 10 g BADG mit Epoxidäquivalentgewicht von 413, 25 g Epoxidnovolak entsprechend Beispiel 1 werden bei einer Temperatur von ca. 200°C gemischt. Nach Abkühlen auf 100°C werden 7 g eines Adduktes aus 2 Mol Phenylglycidyläther und 1 Mol Dimethylaminopropylamin zugemischt und entsprechend Beispiel 1 verarbeitet und gehärtet. Es werden Formkörper mit folgenden Eigenschaften erhalten:

| | |
|---|---|
| BF [MPa] | 111 |
| ε [%] | 3,4. |

Beispiel 12: 60 g Epoxidharz entsprechend Beispiel 10 und 40 g Imid D werden bei ca. 200°C gut gemischt, auf 100°C abgekühlt und mit 6 g Katalysator 1 vernischt. Bei Verarbeitung und Härtung entsprechend Beispiel 1 werden Formkörper mit folgenden Eigenschaften erhalten:

| | |
|---|---|
| Tg [°C] | 175 |
| BF [MPa] | 91. |

Beispiel 13: 15 g Imid E werden mit 40 g Epoxidnovolak entsprechend Beispiel 1, 35 g Imid C und 10 g einer Epoxidverbindung folgender Struktur:

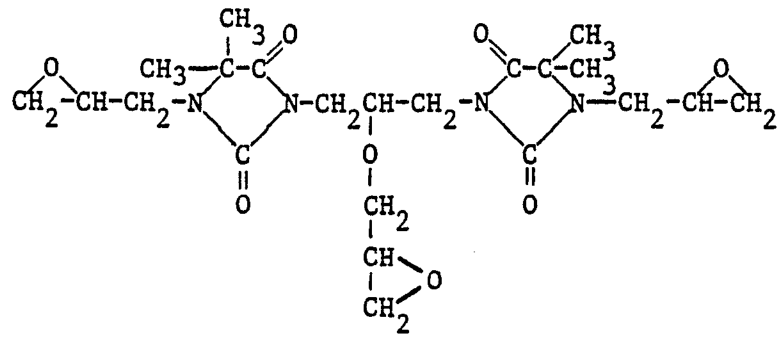

bei ca. 200°C gut gemischt. Nach Abkühlen auf 80°C werden 3 g Phenylimidazol zugegeben. Die Mischung hat eine Gelierzeit von 7 Minuten bei 120°C. Nach Härtung entsprechend Beispiel 1 wird ein Formkörper mit einer Tg von 199°C erhalten.

Beispiel 14: 50 g Epoxidverbindung entsprechend Beispiel 1 werden mit 50 g Imid E bei ca. 200°C gemischt. Nach Abkühlen auf 120°C werden 2,25 g Katalysator 1 zugesetzt. Nach Verarbeitung und Härtung entsprechend Beispiel 1 werden Formkörper mit folgenden Eigenschaften erhalten:

| | |
|---|---|
| BF [MPa] | 125 |
| ε [%] | 8,2. |

## Patentansprüche

1. Lagerstabiles, heisshärtbares Gemisch, enthaltend (a) 5 bis 95 Gewichtsteile eines Epoxidharzes mit durchschnittlich mehr als einer Epoxidgruppe im Molekül,

(b) 95 bis 5 Gewichtsteile eines allyl- oder methallylsubstituierten Imids der Formel I

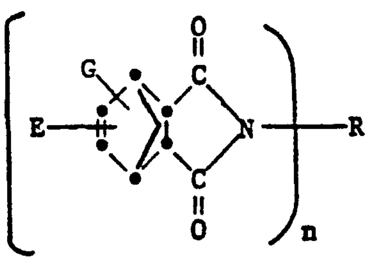

(I),

worin E Allyl oder Methallyl, G Wasserstoff oder Methyl, und n 1 oder 2 bedeuten und R, falls n 1 bedeutet, für Wasserstoff, Alkyl mit 1-12 C-Atomen, Alkenyl mit 3-6 C-Atomen, Cycloalkyl mit 5-8 C-Atomen, Aryl mit 6-10 C-Atomen Hydroxyphenyl oder Benzyl oder, falls n 2 bedeutet, für $-C_mH_{2m}-$ mit m = 2-20, Arylen mit 6-10 C-Atomen oder für eine Gruppe der Formel II

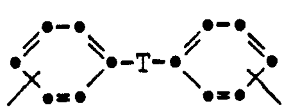

(II),

worin T Methylen, Isopropyliden, CO, O, S oder $SO_2$ bedeutet, steht, und wobei im Gemisch die Summe aus (a) und (b) 100 Gewichtsteile beträgt, und

(c) 1 bis 15 Gewichtsteile, bezogen auf 100 Gewichtsteile des Gemisches aus (a) und (b), eines Härtungskatalysators.

2. Gemisch gemäss Anspruch 1, worin G in Formel 1 Wasserstoff bedeutet.

3. Gemisch gemäss Anspruch 1 oder 2, enthaltend 20 bis 70 Gewichtsteile des Epoxidharzes (a), 80 bis 30 Gewichtsteile des Imids (b) und 3 bis 10 Gewichtsteile des Katalysators (c).

4. Gemisch gemäss Anspruch 1 oder 2, enthaltend als Epoxidharz (a) einen Polyglycidyläther oder ein Poly-(N-glycidyl)-derivat eines aromatischen Amins.

5. Gemisch gemäss Anspruch 1 oder 2, enthaltend als Epoxidharz (a) einen Polyglycidyläther eines mehrkernigen Phenols.

6. Gemisch gemäss Anspruch 1, enthaltend ein Imid der Formel I, worin E Allyl und G Wasserstoff bedeuten und R, falls n = 1 ist, für Wasserstoff, Alkyl mit 1-8 C-Atomen, Allyl, Cyclohexyl, Phenyl, Hydroxyphenyl oder Benzyl steht, oder, falls n = 2 ist, für $-(CH_2)_m-$ mit m = 2-12, 1m- oder p-Phenylen oder für eine Gruppe der Formel II steht, worin T die Methylengruppe, O oder $SO_2$ bedeutet.

7. Gemisch gemäss Anspruch 1, enthaltend ein Imid der Formel I, worin E Allyl, G Wasserstoff, n die Zahl 2 und R $-(CH_2)_2$-$(CH_2)_6$-oder

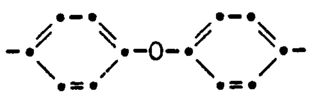 und insbesondere 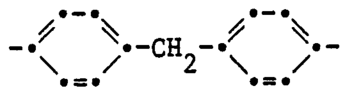

bedeuten.

8. Gemisch gemäss Anspruch 1, enthaltend als Katalysator (c) Imidazol oder dessen substituierte Derivate.

9. Gemische gemäss Anspruch 1, enthaltend als Katalysator (c) ein N-acylsubstituiertes Imidazol.

10. Die aus den härtbaren Gemischen gemäss Anspruch 1 durch Härtung erhaltenen Formstoffe, Beschichtungen oder Verklebungen.

**Claims**

1. A heat-curable mixture which is stable on storage and contains

(a) 5 to 95 parts by weight of an epoxide resin having on average more than one epoxide group in the molecule,

(b) 95 to 5 parts by weight of an allyl-substituted or methallyl-substituted imide of the formula I

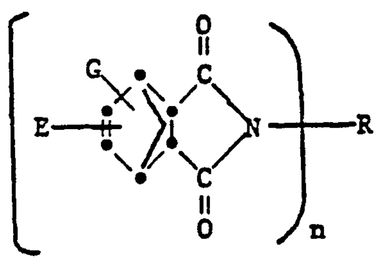

(I)

in which E is allyl or methallyl, G is hydrogen or methyl and n is 1 or 2 and if n is 1 R is hydrogen, alkyl having 1-12 C atoms alkenyl having 3-6 C atoms, cycloalkyl having 5-8 C atoms, aryl having 6-10 C atoms, hydroxyphenyl or benzyl or, if n is 2, R is $-C_mH_{2m}-$ in which m = 2-20, arylene having 6-10 C atoms or a group of the formula II

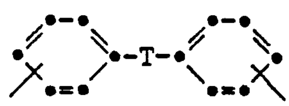

(II)

in which T is methylene, isopropylidene, CO, O, S or $SO_2$, and in which mixture the total of (a) and (b) is 100 parts by weight, and

(c) 1 to 15 parts by weight, based on 100 parts by weight of the mixture of (a) and (b), of a curing catalyst.

2. A mixture according to claim 1, wherein G in formula I is hydrogen.

3. A mixture according to either of claims 1 or 2, which contains 20 to 70 parts by weight of the epoxide resin (a), 80 to 30 parts by weight of the imide (b) and 3 to 10 parts by weight of the catalyst (c).

4. A mixture according to either of claims 1 or 2, which contains, as the epoxide resin (a), a polyglycidyl ether or aa poly-(N-glycidyl) derivative of an aromatic amine.

5. A mixture according to either of claims 1 or 2, which contains, as the epoxide resin (a), a polyglycidyl ether of a polynuclear phenol.

6. A mixture according to claim 1, which contains an imide of the formula I in which E is allyl and G is hydrogen and, if n is 1, R is hydrogen, alkyl having 1-8 C atoms, allyl, cyclohexyl, phenyl, hydroxyphenyl or benzyl, or, if n is 2, R is $-(CH_2)_m-$ in which m is 2-12, m-phenylene or p-phenylene or a group of the formula II in which T is the methylene group, O or $SO_2$.

7. A mixture according to claim 1, which contains an imide of the formula I in which E is allyl, G is hydrogen, n is the number 2 and R is $-(CH_2)_2-$, $-(CH_2)_6-$ or

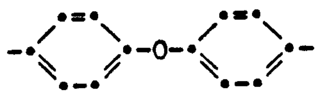 or, in particular, 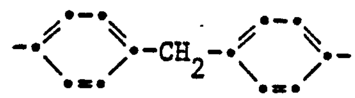 .

8. A mixture according to claim 1, which contains, as the catalyst (c), imidazole or substituted derivatives thereof.

9. A mixture according to claim 1, containing, as the catalyst (c), an N-acyl-substituted imidazole.

10. The moulded materials, coatings or adhesive bonds obtained from the curable mixtures according to claim 1 by curing.

## Revendications

1. Mélange thermodurcissable, stable au stockage, qui contient:

(a) de 5 à 95 parties en poids d'une résine époxydique renfermant en moyenne plus d'un radical époxy par molécule,

(b) de 95 à 5 parties en poids d'un imide porteur d'un radical allyle ou méthallyle qui répond à la formule I:

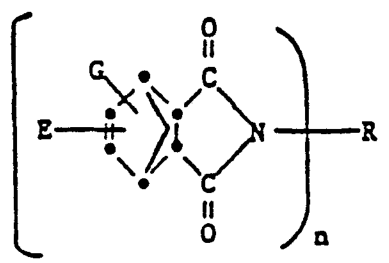

(I)

dans laquelle:

E représente un radical allyle ou méthallyle,

G représente l'hydrogène ou un méthyle,

n est égal à 1 ou à 2 et

R représente, dans le cas où n est égal à 1, l'hydrogène, un alkyle en $C_1$-$C_{12}$, un alcényle en $C_3$-$C_6$, un cycloalkyle en $C_5$-$C_8$, un aryle en $C_6$-$C_{10}$, un hydroxyphényle ou un benzyle et, dans le cas où n est égal à 2, un radical $-C_mH_{2m}-$ dont l'indice m peut aller de 2 à 20, un arylène en $C_6$-$C_{10}$ ou un radical répondant à la formule II:

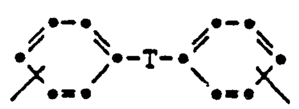

(II)

dans laquelle T représente un radical méthylène, isopropylidène, CO, O, S ou $SO_2$,

la somme des constituants (a) et (b) du mélange représentant 100 parties en poids, et

(c) de 1 à 15 parties en poids, pour 100 parties en poids du mélange de (a) et (b), d'un catalyseur de durcissement.

2. Mélange selon la revendication 1 qui contient un imide de formule I dans lequel G représente l'hydrogène.

3. Mélange selon l'une des revendications 1 et 2, qui contient de 20 à 70 parties en poids de la résine époxydique (a), de 80 à 30 parties en poids de l'imide (b) et de 3 à 10 parties en poids du catalyseur (c).

4. Mélange selon l'une des revendications 1 et 2, qui contient, comme résine époxydique (a), un éther polyglycidylique ou un dérivé poly-(N-glycidylique) d'une amine aromatique.

5. Mélange selon l'une des revendications et 2, qui contient, comme résine époxydique (a) un éther polyglycidylique d'un phénol à plusieurs noyaux.

6. Mélange selon la revendication 1 qui contient un imide de formule 1 dans lequel E représente un radical allyle, G représente l'hydrogène et R représente, lorsque n est égal à I, l'hydrogène, un alkyle en $C_1$-$C_8$, un allyle, un cyclohexyle, un phényle, un hydroxyphényle ou un benzyle et, lorsque n est égal à 2, un radical $-(CH_2)_m-$ dont l'indice n est un nombre de 2 à 12, un m-phénylène, un pphénylène ou un radical de formule II dans lequel T désigne un radical méthylène, O ou $SO_2$.

7. Mélange selon la revendication 1 qui contient un imide de formule I dans lequel E représente un allyle, G l'hydrogène, n le nombre 2 et R un radical $-(CH_2)_2$,

$-(CH_2)_6-$, 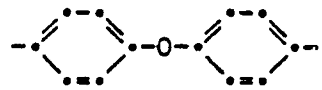 ou, mieux, 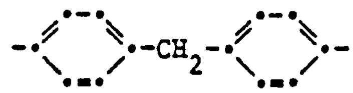 .

8. Mélange selon la revendication 1 qui contient, comme catalyseur (c), de l'imidazole ou l'un de ses dérivés de substitution.

9. Mélange selon la revendication 1 qui contient, comme catalyseur (c), un imidazole acylé à l'azote.

10. Matières moulées, revêtements ou collages qui ont été obtenus par durcissement à partir des mélanges durcissables selon la revendication 1.